(19) Europäisches Patentamt
European Patent Office
Office européen des brevets

(11) EP 0 502 392 B1

(12) **EUROPÄISCHE PATENTSCHRIFT**

(45) Veröffentlichungstag und Bekanntmachung des Hinweises auf die Patenterteilung:
**02.10.1996 Patentblatt 1996/40**

(51) Int Cl.6: **C07D 495/04**, C07F 1/02, C07F 3/02, A61K 31/415 // (C07D495/04, 333:00, 235:00)

(21) Anmeldenummer: **92103040.9**

(22) Anmeldetag: **24.02.1992**

(54) **Verfahren zur Herstellung von D-(+)-Biotin und Zwischenprodukte in diesem Verfahren**

Process for the preparation of D-(+)-biotin and intermediates in this process

Procédé pour la préparation de D-(+)-biotine et intemédiaires dans ce procédé

(84) Benannte Vertragsstaaten:
**CH FR GB IT LI**

(30) Priorität: **06.03.1991 DE 4107121**

(43) Veröffentlichungstag der Anmeldung:
**09.09.1992 Patentblatt 1992/37**

(73) Patentinhaber: **MERCK PATENT GmbH**
**D-64271 Darmstadt (DE)**

(72) Erfinder:
- **Schwarz, Michael, Dr.**
  **W-6080 Gross-Gerau (DE)**
- **Casutt, Michael, Dr.**
  **W-6148 Heppenheim (DE)**
- **Eckstein, Jürgen**
  **W-6101 Rossdorf (DE)**

(56) Entgegenhaltungen:
**EP-A- 0 154 225** **EP-A- 0 242 686**

- **CHEMICAL ABSTRACTS, vol. 70, no. 5, 3. Februar 1969, Columbus, Ohio, US; abstract no. 19984R, I. ISAKA ET AL.: 'Biotin synthesis. IV. A new synthesis of biotin by Grignard reaction of 1,4-butylenedimagnesium halide' Seite 1990 ;**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 502 392 B1

## Beschreibung

Die vorliegende Erfindung betrifft ein neues Verfahren zur Herstellung von heterocyclischen Verbindungen, welche als Zwischenprodukte für die Herstellung von D-(+)-Biotin geeignet sind, sowie ein Verfahren zur Herstellung von D-(+)-Biotin selbst. Die Erfindung betrifft ferner neue Zwischenprodukte in diesem Verfahren.

D-(+)-Biotin ist eine seit langem bekannte Substanz, und es sind demgemäß auch bereits eine Reihe Verfahren zu deren Herstellung bekannt. Den technisch interessanten Verfahren ist die Notwendigkeit gemeinsam, auf irgendeiner Stufe die Carboxybutylseitenkette an das Ringsystem anzuhängen. Hierfür sind verschiedene Lösungen bekannt, wie etwa der Aufbau der Seitenkette nach dem Verknüpfungsschema $C_4 + C_1 \rightarrow C_5$ oder auch $C_3 + (C_3\text{-}C_1 = C_2) \rightarrow C_5$ (z. B. CH-PS 556/867).

Es ist auch bekannt, die Seitenkette in einer Stufe mittels einer Wittig-Reaktion mit dem Ringsystem zu verknüpfen (z.B. EP-OS-0 084 377). Alle diese Verfahren haben jedoch den Nachteil, daß sie entweder über eine verhältnismäßig große Anzahl von Reaktionsstufen verlaufen oder aber einen relativ großen Aufwand zur Isolierung des gewünschten Endproduktes erfordern.

Weiterhin ist aus der EP-OS-0 154 225 die Einführung der Seitenkette durch Umsetzung des Thiolactons der Formel I mit 4-(2.4.10-Trioxaadamantyl)-butylmagnesiumbromid bekannt.

Diese Verfahren weist jedoch verschiedene Nachteile auf, welche es bei einer technischen Durchführung als ungeeignet erscheinen lassen.

Das zur Herstellung des Grignard-Reagenz benötigte cis-1,3,5-Cyclohexantriol ist nur schlecht zugänglich und muß nach erfolgter Umsetzung durch technisch aufwendige Isolierungstechniken wieder zurückgewonnen werden. Außerdem sind die Ausbeuten bei diesem Verfahren nicht ausreichend für eine industrielle Ausnutzung.

Es bestand somit ein Bedürfnis nach einem technisch einfachen Verfahren, gemäß welchem die Seitenkette in guter Ausbeute und in möglichst einem Reaktionsschritt an das Ringsystem angehängt werden kann. Dies ist nunmehr mittels des erfindungsgemäßen Verfahrens möglich.

Diese Verfahren ist dadurch gekennzeichnet, daß man das Thiolacton der Formel I,

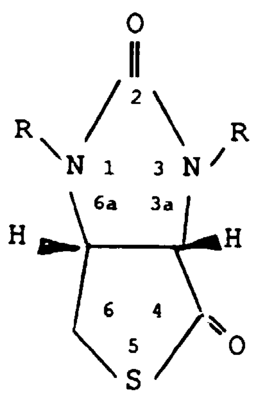

I

worin R eine unsubstituierte oder durch ein oder zwei Alkylgruppen mit 1-5 C-Atomen substituierte Benzylgruppe darstellt, mit einer metallorganischen Verbindung der Formel II,

$$\text{Met-CH}_2\text{-CH}_2\text{-CH}_2\text{-CH}_2\text{-C(OR}^1)_3 \qquad \text{II}$$

worin Met Li, MgCl oder MgBr, und $R^1$ Alkyl mit 1 bis 3 C-Atomen bedeuten, umsetzt, daß man, die so erhaltene Verbindung der Formel III,

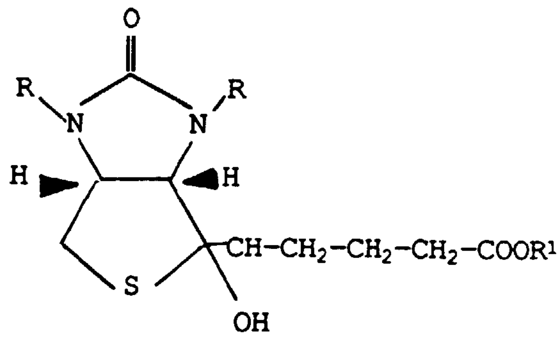

III

worin R und $R^1$ die obige Bedeutung haben, dehydratisiert, daß man, in der so erhaltenen Verbindung der Formel IV,

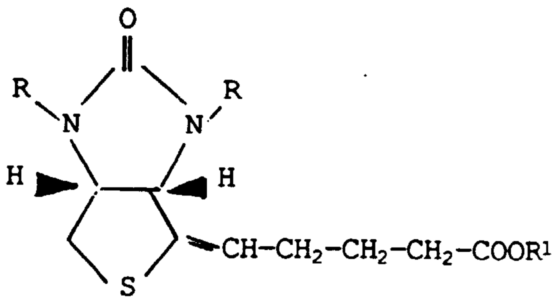

IV

worin R und $R^1$ die obige Bedeutung haben, durch Verseifung, die Carboxylgruppe in der Seitenkette freisetzt, und daß man die so erhaltene Verbindung der Formel V,

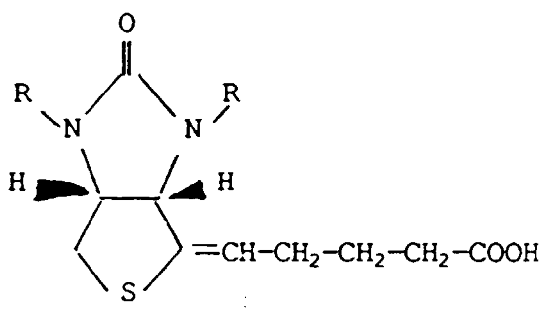

V

worin R die obige Bedeutung hat, in an sich bekannter Weise in D-(+)-Biotin überführt.

Die als Ausgangsmaterialien verwendete Verbindung der Formel I, sowie die erfindungsgemäß hergestellten Verbindungen der Formel IV und V sind bekannte Verbindungen (z.B. EP 0 084 377 R = Benzyl oder EP 0 273 270 R = 1-Phenylethyl). Die Verbindungen der Formel II und III sind jedoch neu und ebenfalls Gegenstand der vorliegenden Erfindung.

Die Herstellung der Verbindungen der Formel II kann in Analogie zu bekannten Verfahren gemäß folgendem Schema durchgeführt werden:

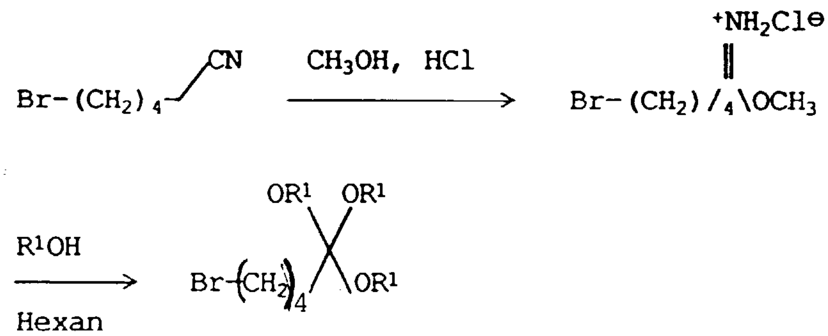


(z.B. G. Caby et al., Org. Synth. 67, 193-201 (1988))

In den Verbindungen der Formeln I bis V bedeutet R vorzugsweise eine unsubstituierte Benzylgruppe oder eine (R oder S)-(1-Phenylethyl)-gruppe und $R^1$ Methyl, Ethyl oder Propyl, insbesondere Methyl.

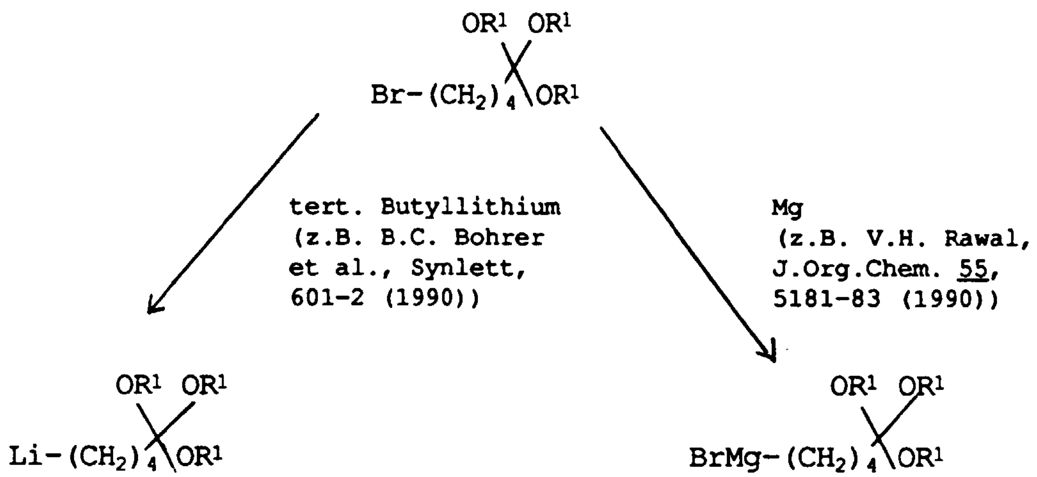


Die Umsetzung des Thiolactons der Formel I mit einer metallorganischen Verbindung der Formel II kann in an sich bekannter Weise, d.h. unter den für eine solche Reaktion üblichen Bedingungen durchgeführt werden. Zweckmäßig erfolgt dies in einem unter den Reaktionsbedingungen inerten organischen Lösungsmittel, beispielsweise in einem niederen Alkylether wie Diethylether oder einem cyclischen Ether wie Tetrahydrofuran, Dioxan und dergleichen und bei einer Temperatur von etwa -78 °C bis zum Siedepunkt des verwendeten Lösungsmittels, vorzugsweise bei etwa 0 °C bis etwa 50 °C, insbesonderer bei Raumtemperatur.

Die Orthoestergruppe wird bei der wäßrigen Aufarbeitung hydrolysiert. Man erhält die Verbindung der Formel III, ohne daß ein zusätzlicher Abspaltungsschritt erforderlich wäre.

Die Dehydratisierung der Verbindung der Formel III kann in an sich bekannter Weise durchgeführt werden. Zweckmäßig erfolgt dies durch Behandlung mit einer Säure wie etwa Schwefelsäure, Salzsäure, p-Toluolsulfonsäure und dergleichen. Als Lösungsmittel wird zweckmäßig ein solches verwendet, welches mit dem gebildeten Wasser ein Azeotrop bildet, z.B. aromatische Kohlenwasserstoffe wie Benzol, Toluol, Xylol unter dergleichen. Die Dehydratisierung erfolgt auch vorteilhaft bei erhöhter Temperatur, vorzugsweise bei Rückflußtemperatur des Reaktionsgemisches.

Die nach der Dehydratisierung erhaltene Verbindung der Formel IV ist, wie bereits erwähnt, eine bekannte Verbindung (z.B. EP OS 084 377) und kann in bekannter Weise, d.h. durch Verseifung, Hydrierung der Doppelbindung und Abspaltung der Schutzgruppen an den Stickstoffatomen leicht in D-(+)-Biotin überführt werden (z.B. CH-PS 556867).

Die nachfolgenden Beispiele erläutern die Erfindung.

Beispiel 1

Synthese von 5-Chlor-imidopentansäure-methylester-hydrochlorid.

24,94 g (210 mMol) 5-Chlor-valeronitril werden in 210 ml absolutem Diethylether und 8,08 g (252 mMol) absolutem

Methanol gelöst. Bei Raumemperatur werden innerhalb 4 h 19,2 g (525 mMol) getrocknetes HCl-Gas eingeleitet. Die Reaktionslösung wird gekühlt und 120 h stehengelassen.

Die ausgefallenen Kristalle werden unter Stickstoff abgesaugt, mit 2 x 100 ml Diethylether und 150 ml n-Hexan gewaschen.

Analog werden hergestellt:

5-Chlor-imidopentansäure-ethylester-hydrochlorid
5-Chlor-imidopentansäure-propylester-hydrochlorid

Beispiel 2

Synthese von 5-Chlor-orthopentansäure-trimethylester.

Unter Stickstoff werden 35,36 g (190 mMol) 5-Chlor-imidopentansäure-methylester-hydrochlorid in 470 ml n-Hexan suspendiert. Anschließend werden 18,3 g (570 mMol) Methanol zugegeben und die Suspension 48 h bei Raumtemperatur heftig gerührt.

Die Reaktionslösung wird vom ausgefallenen Ammoniumchlorid abgesaugt, eingeengt und der Rückstand unter Zusatz von 0,3 g Kaliumcarbonat im Vakkuum destilliert.
Sdp.: 58-59 °C (1,1 Torr)

Analog werden hergestellt:

5-Chlor-orthopentansäure-triethylester
5-Chlor-orthopentansäure-tripropylester

Beispiel 3

Synthese von cis-2-Oxo-1,3-dibenzyl-4-hydroxy-hexahydroxy-1H-thieno[3,4-d]imidazol-4-yl-pentansäure methylester

Unter Stickstoff werden 390 mg (15,9 mMol) Magnesiumspäne in 5 ml THF suspendiert und auf 78 °C erwärmt. 3 ml einer Lösung von 3,0 g (15 mMol) 5-Chlor-orthopentansäure-trimethylester in 10 ml THF und 100 µl 1,2-Dibromethan werden zugetropft.

Nachdem die Reaktion gestartet ist, wird der Rest der Lösung in 5 Minuten zugetropft. Nach 15 Minuten Rückfluß wird die Reaktion mit 10 ml THF verdünnt und weitere 15 Minuten nachgerührt.

Die Reaktionslösung wird zu einer Lösung von 2,59 g (7,8 mMol) (+)-cis-1,3-Dibenzyl-hexahydro-1H-thieno [3,4-d] imidazol-2,4-dion in 15 ml THF zugetropft, dabei steigt die Temperatur auf 38 °C. Die Reaktion wird 3 h nachgerührt.

Nach üblicher Aufarbeitung und Chromatographie erhält man 3,19 g (90 % d.Th.) cis-2-Oxo-1,3-dibenzyl-4-hydroxy-hexahydro-1H-thieno[3,4-d]imidazol-4-ylpentansäuremethylester.

Analog werden hergestellt:

cis-2-Oxo-1,3-dibenzyl-4-hydroxy-hexahydro-1H-thieno-[3,4-d]imidazol-4-ylpentansäureethylester
cis-2-Oxo-1,3-dibenzyl-4-hydroxy-hexahydro-1H-thieno-[3,4-d]imidazol-4-ylpentansäurepropylester
cis-2-Oxo-1-[(R)-(1-phenylethyl)]-3-benzyl-4-hydroxy-hexahydro-1H-thieno-[3,4-d]imidazol-4-ylpentansäurepropylester

Beispiel 4

Synthese von cis-2-Oxo-1,3-dibenzylhexahydro-1H-thieno [3,4-d]imidazol-4-yliden-pentansäuremethylester

Zu einem Gemisch aus 3,41 g (7,5 mMol) cis-2-Oxo-1,3-dibenzyl-4-hydroxy-hexahydro-1H-thieno[3,4-d]imidazol-4-ylpentansäuremethylester (hergestellt nach Beispiel 3) und 40 ml THF werden 15 ml 30%ige Schwefelsäure zugetropft und für 30 Minuten auf 55 °C erwärmt.

Nach dem üblichen Aufarbeiten und Säulenchromatographie mit Toluol/Ethylacetat 7:3 erhält man 2,94 g cis-2-Oxo-1,3-dibenzylhexahydro-1H-thieno[3,4-d] imidazol-4-ylidenpentansäuremethylester (90 % d.Th.), $[\alpha]^{25}_{D}$ = +230,4 (c = 1, Benzol)

Analog werden hergestellt:

cis-2-Oxo-1,3-dibenzyl-hexahydro-1H-thieno[3,4-d]imidazol-4-ylidenpentansäureethylester
cis-2-Oxo-1,3-dibenzyl-hexahydro-1H-thieno[3,4-d]imidazol-4-ylidenpentansäurepropylester

cis-2-Oxo-1-[(R)-(1-phenylethyl)]-3-benzyl-4-hydroxy-hexahydro-1H-thieno-[3,4-d]imidazol-4-ylpentansäurepropylester

**Patentansprüche**

1. Verfahren zur Herstellung von D-(+)-Biotin, dadurch gekennzeichnet, daß man das Thiolacton der Formel I,

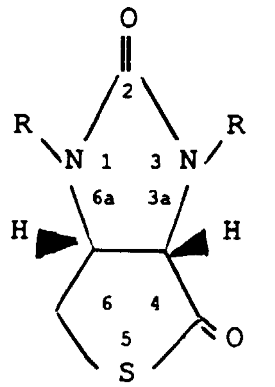

I

worin R eine unsubstituierte oder durch eine oder zwei Alkylgruppen mit 1 bis 5 C-Atomen substituierte Benzylgruppe darstellt, mit einer metallorganischen Verbindung der Formel II,

$$Met\text{-}CH_2\text{-}CH_2\text{-}CH_2\text{-}CH_2\text{-}C(OR^1)_3 \quad \text{II}$$

worin Met Li, MgCl oder MgBr, und $R^1$ Alkyl mit 1 bis 3 C-Atomen bedeuten,
umsetzt, daß man die so erhaltene Verbindung der Formel III,

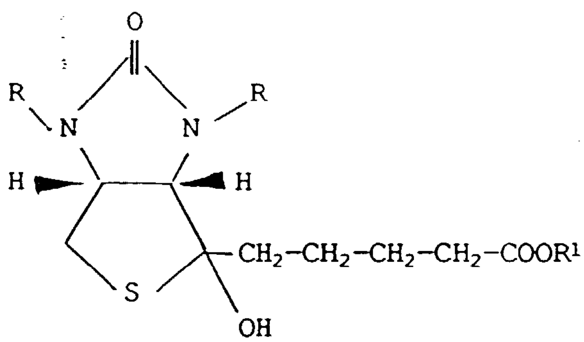

III

worin R und $R^1$ die obige Bedeutung haben, dehydratisiert, daß man in der so erhaltenen Verbindung der Formel IV,

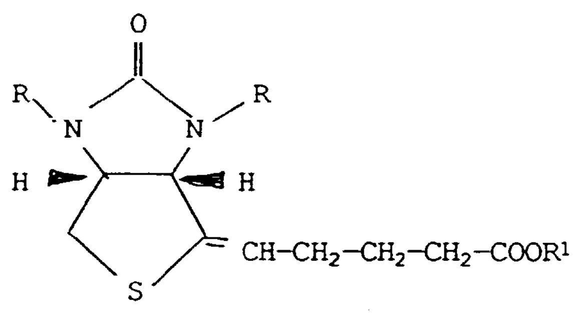

IV

worin R und $R^1$ die obige Bedeutung haben, durch Verseifung die Carboxylgruppe der Seitenkette freisetzt, und daß man die so erhaltene Verbindung der Formel V,

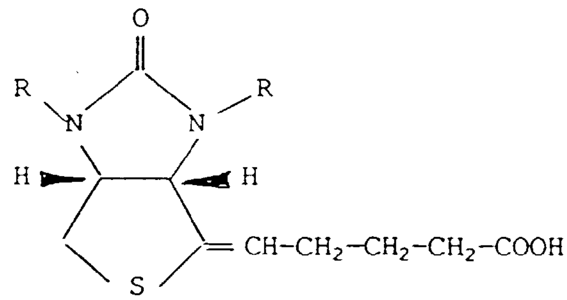

V

worin R die obige Bedeutung hat, in an sich bekannter Weise in D-(+)-Biotin überführt.

2. Verfahren nach Anspruch 1, dadurch gekennzeichnet, daß man die Dehydratisierung mit einer Mineralsäure durchführt.

3. Verfahren nach Anspruch 2, dadurch gekennzeichnet, daß man als Mineralsäure Schwefelsäure, Chlorwasserstoffsäure oder Bromwasserstoffsäure verwendet

4. Die Verbindung der Formel III,

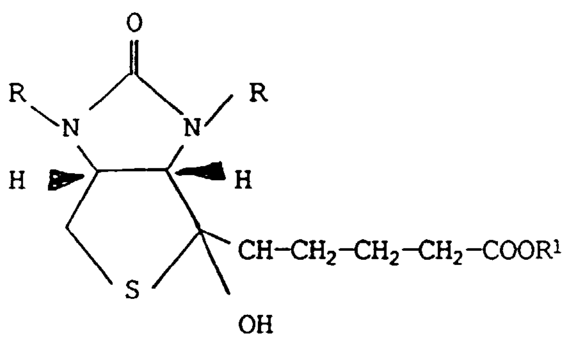

III

worin R eine unsubstituierte oder durch eine oder zwei Alkylgruppen mit 1 bis 5 C-Atomen substituierte Benzylgruppe, vorzugsweise eine unsubstiuierte Benzylgruppe darstellt und $R^1$ den Rest der Formel $CH_3$ bedeutet.

5. Die Verbindung der Formel

$$Met\text{-}CH_2\text{-}CH_2\text{-}CH_2\text{-}CH_2\text{-}C(OR^1)_3 \qquad II$$

worin Met Li, MgBr oder MgCl, und $R^1$ Alkyl mit 1 bis 3 C-Atomen bedeutet.

## Claims

1. Process for the preparation of D-(+)-biotin, characterised in that the thiolactone of the formula I

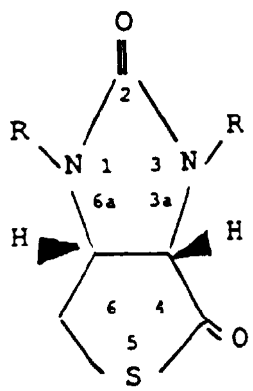

I

in which R is a benzyl group which is unsubstituted or substituted by one or two alkyl groups having 1 to 5 C atoms, is reacted with an organometallic compound of the formula II

$$Met\text{-}CH_2\text{-}CH_2\text{-}CH_2\text{-}CH_2\text{-}C(OR^1)_3 \qquad II$$

in which Met is Li, MgCl or MgBr, and $R^1$ is alkyl having 1 to 3 C atoms, in that the compound thus obtained of the formula III

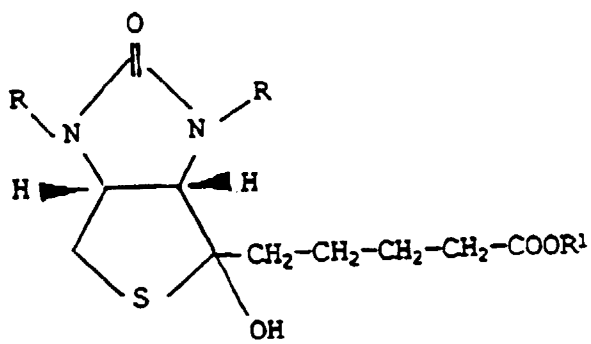

III

in which R and $R^1$ have the above meaning, is dehydrated, in that in the compound thus obtained of the formula IV

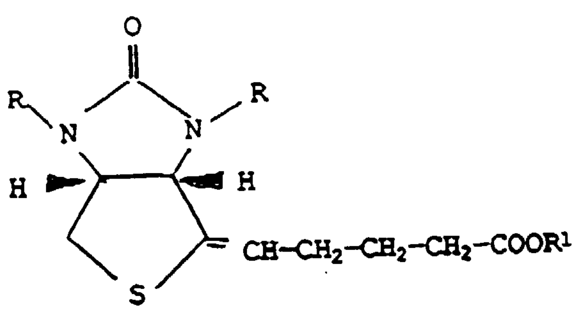

IV

in which R and $R^1$ have the above meaning, the carboxyl group of the side chain is liberated by alkaline hydrolysis, and in that the compound thus obtained of the formula V

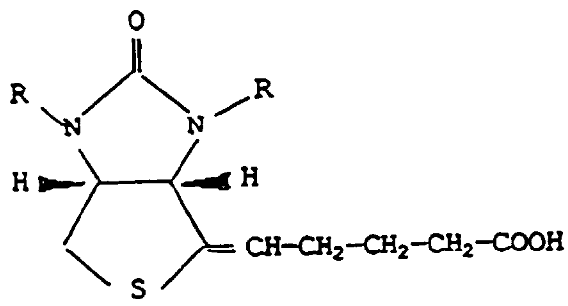

V

in which R has the above meaning, is converted into D-(+)-biotin in a manner known per se.

2. Process according to Claim 1, characterised in that the dehydration is carried out using a mineral acid.

3. Process according to Claim 2, characterised in that sulfuric acid, hydrochloric acid or hydrobromic acid is used as the mineral acid.

4. The compound of the formula III

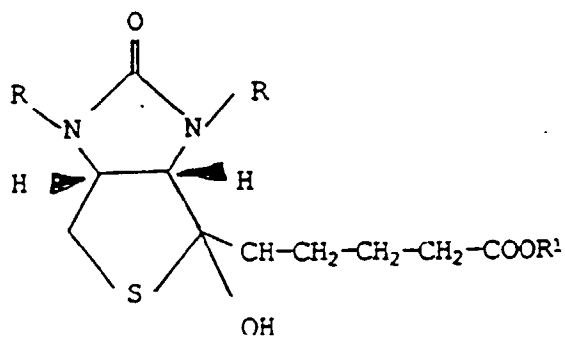

III

in which R is a benzyl group which is unsubstituted or substituted by one or two alkyl groups having 1 to 5 C atoms, preferably an unsubstituted benzyl group, and $R^1$ is the radical of the formula $CH_3$.

5. The compound of the formula

$$Met\text{-}CH_2\text{-}CH_2\text{-}CH_2\text{-}CH_2\text{-}C(OR^1)_3 \qquad II$$

in which Met is Li, MgBr or MgCl, and $R^1$ is alkyl having 1 to 3 C atoms.

## Revendications

1. Procédé de préparation de D-(+)-biotine, caractérisé en ce qu'on fait réagir la thiolactone de formule I,

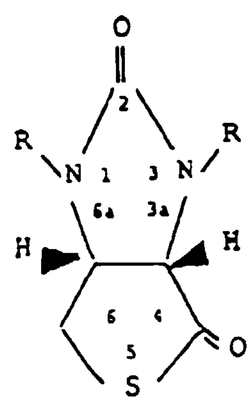

I

dans laquelle R représente un groupe benzyle non substitué ou substitué par un ou deux groupes alkyle en $C_1$ à $C_5$, avec un composé organo-métallique de formule II,

$$Met\text{-}CH_2\text{-}CH_2\text{-}CH_2\text{-}CH_2\text{-}C(OR^1)_3 \qquad II$$

dans laquelle Met représente Li, MgCl ou MgBr, et $R^1$ représente un alkyle en $C_1$ à $C_3$, en ce qu'on déshydrate le composé de formule III ainsi obtenu,

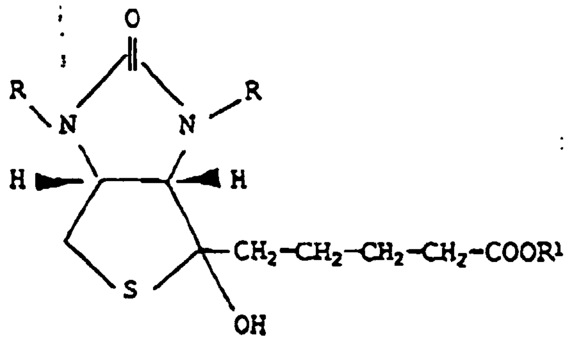

III

dans laquelle R et $R^1$ ont la signification donnée ci-dessus, en ce que dans le composé de formule IV ainsi obtenu,

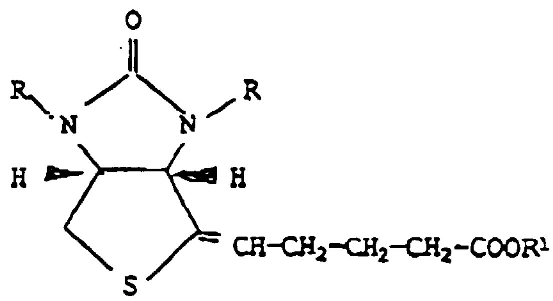

IV

dans laquelle R et $R^1$ ont la signification donnée ci-dessus, on libère par saponification le groupe carboxyle de la chaîne latérale, et en ce qu'on transforme de façon connue en D-(+)-biotine le composé de formule V ainsi obtenu,

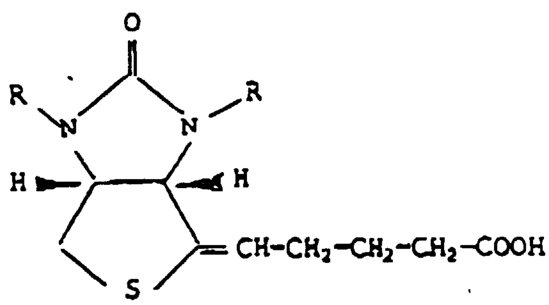

V

dans laquelle R a la signification donnée ci-dessus.

**2.** Procédé selon la revendication 1, caractérisé en ce qu'on procède à la déshydratation avec un acide minéral.

**3.** Procédé selon la revendication 2, caractérisé en ce qu'on utilise comme acide minéral l'acide sulfurique, l'acide chlorhydrique ou l'acide bromhydrique.

**4.** Composé de formule III,

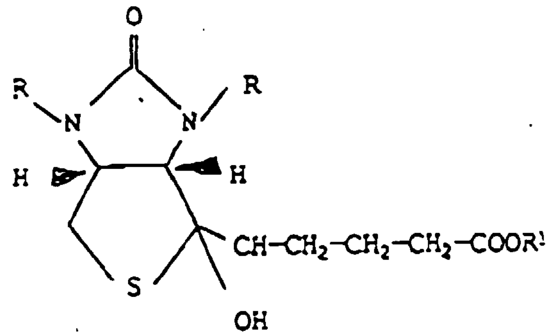

III

dans laquelle R représente un groupe benzyle non substitué ou substitué par un ou deux groupes alkyle en $C_1$ à $C_5$, de préférence un groupe benzyle non substitué et $R^1$ représente un radical de formule $CH_3$.

**5.** Composé de formule

$$Met\text{-}CH_2\text{-}CH_2\text{-}CH_2\text{-}CH_2\text{-}C(OR^1)_3 \quad II$$

dans laquelle Met représente Li, MgBr ou MgCl, et $R^1$ représente un alkyle en $C_1$ à $C_3$.